(19) Europäisches Patentamt
European Patent Office
Office européen des brevets

(11) Publication number: **0 462 521 A1**

(12) # EUROPEAN PATENT APPLICATION

(21) Application number: **91109784.8**

(22) Date of filing: **14.06.91**

(30) Priority: **19.06.90 JP 160597/90**
**01.10.90 JP 264580/90**
**08.05.91 JP 102629/91**

(43) Date of publication of application:
**27.12.91 Bulletin 91/52**

(84) Designated Contracting States:
**AT BE CH DE DK ES FR GB GR IT LI LU NL SE**

(51) Int. Cl.5: **C07D 499/88**, A61K 31/43

(71) Applicant: **TAKEDA CHEMICAL INDUSTRIES, LTD.**
**3-6, Doshomachi 2-chome Chuo-ku**
**Osaka 541(JP)**

(72) Inventor: **Maeda, Yoshiharu**
**20-38, Takabedai 1-chome**
**Tondabayashi, Osaka 584(JP)**
Inventor: **Ishibashi, Yukio**
**6-301, 6 Minamisakurazuka 3-chome**
**Toyonaka, Osaka 560(JP)**
Inventor: **Tsukamoto, Tetsuya**
**6-307, 1 Minamikoshien 3-ohome**
**Nishinomiya, Hyogo 663(JP)**

(74) Representative: **Lederer, Franz, Dr. et al**
**Lederer, Keller & Riederer, Patentanwälte,**
**Lucile-Grahn-Strasse 22**
**W-8000 München 80(DE)**

(54) **Crystalline penem, its production and use.**

(57) Crystalline (+)-(5R,6S)-6-[(R)-1-hydroxyethyl]-3-(3-pyridyl)-7-oxo-4-thia-1-azabicyclo[3.2.0]hept-2-ene-2-carboxylic acid pivalcyloxymethyl ester having a diffraction pattern which shows main peaks at spacing of 12.8, 8.8, 5.6, 4.44, 4.36, and 4.2 Å according to powder X-ray diffraction is disclosed. The crystalline penem compound is useful for an antibacterial agent.

EP 0 462 521 A1

## FIELD OF THE INVENTION

The present invention relates to a crystalline penem compound which is useful as an antibacterial compound for medical use, its production and use.

## BACKGROUND OF THE INVENTION

Japanese Patent Laid Open Publication No. 263138/1987 (EP-A 246187) discloses certain 2-pyridylpenem compounds. Among them, (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester of the formula:

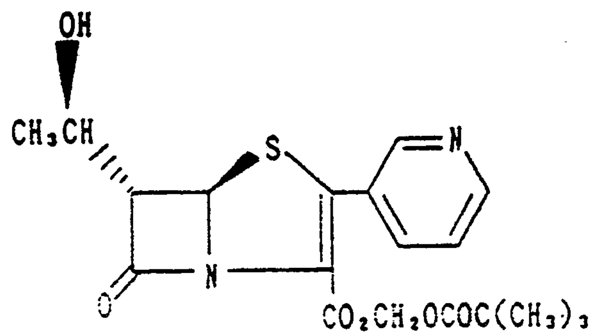

is a particularly useful penem compound which manifests excellent antibacterial activities against not only gram-negative bacteria but also gram-positive bacteria by oral administration, and its practical use has been studied.

However, in spite of its excellent antibacterial activities, the penem compound has been obtained only in an amorphous form. This amorphous solid has a disadvantage that it has insufficient stability and decolors when it is stored for a long period of time under normal conditions, which results in decrease in the content of an active component upon production of its preparations. Further, there is another disadvantage that the complicated purification step is required to obtain a substantially pure amorphous solid.

Under these circumstances, the present inventors have intensively studied to obtain the penem compound having excellent antibacterial activities in a crystalline form having good storage stability to improve the above disadvantages. As a result, it has been found that the penem compound can be obtained as a stable crystalline form, the purification can be easily conducted by crystallization, and further the crystalline penem compound can be obtained from a water-ethanol system which is beneficial as medicines from the viewpoint of a residual solvent in crystals.

## OBJECTS OF THE INVENTION

The main object of the present invention is to provide the penem compound having excellent antibacterial activities in a stable crystalline form.

This object as well as other objects and advantages of the present invention will become apparent to those skilled in the art from the following description with reference to the accompanying drawing.

## BRIEF EXPLANATION OF DRAWING

Fig. 1 is a powder X-ray diffraction pattern of the crystalline (+)-(5R, 6S)-6-[(R)-1-hydroxyethyl]-3-(3-pyridyl)-7-oxo-4-thia-1-azacyclo[3.2.0]hept-2-ene-2-carboxylic acid pivaloyloxymethyl ester of the present invention.

Fig. 2 is a powder X-ray diffraction pattern of the crystalline potassium (+)-(5R, 6S)-6-[(R)-1-hydroxyethyl]-3-(3-pyridyl)-7-oxo-4-thia-1-azabicyclo[3.2.0]hept-2-ene-2-carboxylate.

## SUMMARY OF THE INVENTION

According to the present invention, there is provided crystalline (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester having a diffraction pattern which shows main peaks at spacing of 12.8, 8.8, 5.6, 4.44, 4.36, and 4.2 Å according to powder X-ray diffraction.

The crystalline penem compound is obtained by dissolving amorphous (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester in a good solvent, adding thereto a poor solvent which is miscible with the good solvent, stirring the mixture and then cooling to a temperature of not higher than 30 °C. The present invention also provides this production process as well as an antibacterial agent comprising the crystalline penem compound.

## DETAILED EXPLANATION OF THE INVENTION

The amorphous (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester to be used in the present invention can be obtained by esterifying its sodium salt with methylpivalate iodide according to the disclosure of the above Japanese Patent Laid Open Publication No. 263183/1987.

Examples of the good solvent of the amorphous penem compound include lower alcohols such as methanol, ethanol, propanol, isopropanol and the like and esters such as ethyl acetate and the like. Upon crystallization, if necessary, it is heated up to about 40 °C to dissolve the amorphous penem compound in the good solvent. The concentration of the amorphous penem compound is not specifically limited but, in view of higher yield of the crystal, the concentration is preferably made as high as possible. In the present invention, a reaction mixture from the esterification step in the production of the penem compound may be used directly without isolation of the amorphous penem compound by diluting the reaction mixture with the good solvent, purified and then optionally concentrated, preferably, under reduced pressure.

Examples of the poor solvent which is miscible with the good solvent include water in the case of using an alcohol as the good solvent as well as ethyl ether, n-hexane, petroleum ether and the like in the case of using an ester as the good solvent. The poor solvent is added in an amount sufficient for crystallization of the penem compound. Normally, the volume ratio of the good solvent to the bad solvent is about 1 : 1.5 to 10.

When the mixture of the poor solvent and good solvent containing the penem compound is stirred at a temperature of, normally, room temperature to 35 °C (-10 to 35 °C) for 5 minutes to 2 hours, the crystallization of the penem compound is started. In this case, the crystallization can be promoted, for example, by rubbing the wall of a container or the like according to a conventional method. Then, it is cooled to 30 °C or lower, preferably -5 to 15 °C and, further, stirred for 30 minutes to 20 hours, or allowed to stand to complete crystallization. The resulting crystals are washed, dried and then isolated according to a conventional method.

The crystalline penem compound thus obtained according to the present invention has a melting point of about 95 to 96 °C and shows a diffraction pattern having main peaks at spacing of 12.8, 8.8, 5.6, 4.44, 4.36 and 4.2 Å according to powder X-ray diffraction.

The crystalline penem compound is useful as an antibiotic having broad antibacterial spectrum by oral administration. For example, the penem compound have a minimum inhibitory concentration of about 8 µg/ml or less against both gram-positive and gram-negative bacteria such as Staphylococcus aureus, Staphylococcus pyogenes, Streptococcus pneumoniae, Streptococcus faecalis and bacteria of the genus Neisseria. In addition, it has a minimum inhibitory concentration of about 64 µl/ml or less against gram-negative rod bacteria such as those of the genus Enterobacter, Haemophilus influenzae, bacteria of the genus Pseudomonas and the like and aerobic bacteria such as the genus Bacteroides. Further, in mouse systematic infection caused by Streptococcus aureus, it has $ED_{50}$ of about 0.5 to 15 mg/kg by oral administration.

Therefore, the crystalline penem compound of the present invention can be used as an agent for treatment and prevention of bacterial infectious diseases of human and other mammal, for example, respiratory infectious diseases, urinary tract infectious diseases, suppurative diseases, biliary infectious diseases, enteral infectious diseases, obstetrics and gynecology infectious diseases, otorhinology infectious diseases, surgery infectious diseases and the like.

For oral administration, the crystals are formulated in the form of tablets or capsules according to a conventional method, which can contain various additives, for example, diluents such as lactose, glucose, sucrose, mannitol, sorbitol, cellulose, glycine, etc.; lubricants such as silica, talc, stearic acid or its salt, polyethylene glycol, etc.; binders such as aluminum magnesium silicate, starch, gelatin, gums, cellulose derivatives, polypyrrolidone, etc.; disintegrators such as starch, alginic acid or its salt, etc.; colorants; flavors; sweeteners and the like.

The dosage varies depending upon a particular patient, conditions of diseases and the like. Usually, for an adult patient of 70 kg in body weight, the desired effect can be obtain by orally administration of about 50 mg to 1 g per day.

As described above, according to the present invention, crystalline (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester having excellent antibacterial activity and storage stability can be obtained, particularly, from a water-ethanol system which is beneficial from the viewpoint of the residual solvent. Therefore, the crystalline penem compound is an extremely useful medicine.

The present invention further provides a novel penem compound of the formula (I):

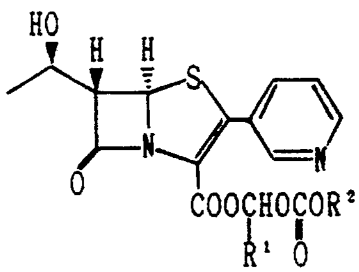

( I )

wherein $R^1$ is hydrogen atom or a lower alkyl; and $R^2$ is an alkyl group branched at a position or a cycloalkyl. The penem compound (I) also manifests excellent antibacterial activities against not only gram negative bacteria but also gram-positive bacteria by oral administration and has good properties for medicines such as good absorbability, low toxicity, good stability and the like.

In the compound (I), examples of the lower alkyl group represented by $R^1$ include $C_{1-6}$ alky such as methyl, ethyl, propyl, isopropyl and the like. A preferred example of the alkyl of $R^1$ is methyl or ethyl.

Examples of the alkyl group branched at α position represented by $R^2$ include $C_{3-10}$ alkyl branched at α position such as isopropyl, sec-butyl, 1-ethylpropyl, 1-methylbutyl, 1-ethylbutyl, 1-methylpentyl, 1-propylbutyl, 1-butylpentyl and the like. A preferred example of the alkyl group branched at a position of $R^2$ is isopropyl. Examples of the cycloalkyl group represented by $R^2$ include $C_{3-10}$ cycloalkyl such as cyclopropyl, cyclobutyl, cyclopentyl, cyclohexyl, cycloheptyl, 2-methylcyclohexyl, 3-methylcyclohexyl, 2-isopropyl-5-methylcyclohexyl and the like. A preferred example of the cycloalkyl of $R^2$ is cyclohexyl.

The compound (I) can be produced by reacting (+)-(5R, 6S)-6-[(R)-1-hydroxyethyl]-3-(3-pyridyl)-7-oxo-4-thia-1-azabicyclo[3.2.0]hept-2-ene-2-carboxylic acid or its salt (e.g. a salt with an alkali metal such as sodium, potassium, etc.) with a compound of the formula (II):

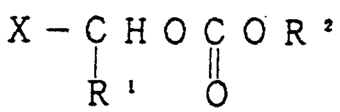

( II )

wherein $R^1$ and $R^2$ are as defined above and X is a halogen atom such as iodine, bromine, chlorine, fluorine or the like.

Usually, this reaction is conducted in an organic solvent. Any organic solvent can be used in so far as it does not adversely effect on the reaction. Examples of the organic solvent include hydrocarbons such as hexane, benzene, toluene, xylene and the like, ethers such as tetrahydrofuran, isopropyl ether, dioxane, diethyl ether and the like, halogenated hydrocarbons such as methylene chloride, carbon tetrachloride and the like, esters such as ethyl acetate and the like, ketones such as acetone and the like, amides such as N,N-dimethylformamide, N,N-dimethylacetamide and the like, and a mixture thereof. Preferred examples of the solvent are amide such as N,N-dimethylformamide, N,N-dimethylacetamide and the like. The reaction temperature is not specifically limited in so far as the esterification reaction proceeds. Usually, the reaction is conducted at -50 to 80°C, preferably, -30 to 30°C. The reaction time varies depending upon the particular compound (II), solvent, reaction temperature and the like but, preferably, the reaction is conducted within about several hours, more preferably, for 1 to 30 minutes. The amount of the compound (II) to be used is normally about 1 to 2 moles, preferably, 1 to 1.5 moles based on 1 mole of (+)-(5R, 6S)-6-[(R)-1-hydroxyethyl]-3-(3-pyridyl)-7-oxo-4-thia-1-azabicyclo[3.2.0]hept-2-ene-2-carboxylic acid or its salt.

It has been found that the potassium salt of (+)-(5R, 6S)-6-[(R)-1-hydroxyethyl]-3-(3-pyridyl)-7-oxo-4-thia-1-azabicyclo[3.2.0]hept-2-ene-2-carboxylic acid is very useful for the production of the compound (I) in comparison with the corresponding known sodium salt because the sodium salt powder is hardly crystallized, while the potassium salt is readily crystallized and, upon producing the compound (I), it is superior in reactivity as well as the yield and purity of the desired ester compound (I) and the like to the sodium salt.

The compound (I) thus obtained can be isolated and purified by known means such as solvent extraction, conversion of liquid properties, concentration, crystallization, recrystallization, chromatography and the like.

The starting compound used in the production of the compound (I), (+)-(5R, 6S)-6-[(R]-1-hydroxyethyl]-3-(3-pyridyl)-7-oxo-4-thia-1-azabicyclo[3.2.0]hept-2-ene-2-carboxylic acid or its salt, can be produced by, for example, the process disclosed in the above Japanese Patent Laid Open publication No. 263138/1987 or a modification thereof. The compound (II) can be produced by, for example, the process disclosed in J. Antibiotics, 40, 81-90 (1987); and J. Antibiotics, 40, 370-384 (1987), or a modification thereof.

The preferred examples of the compound (I) are as follows:

(+)-(5R, 6S)-6-[(R)-1-hydroxyethyl]-3-(3-pyridyl)-7-oxo-4-thia-1-azabicyclo[3.2.0]hept-2-ene-2-carboxylic acid 1-(cyclohexyloxycarbonyloxy)ethyl ester,

(+)-(5R, 6S)-6-[(R)-1-hydroxyethyl]-3-(3-pyridyl)-7-oxo-4-thia-1-azabicyclo[3.2.0]hept-2-ene-2-carboxylic acid cyclohexyloxycarbonyloxymethyl ester,

(+)-(5R, 6S)-6-[(R)-1-hydroxyethyl]-3-(3-pyridyl)-7-oxo-4-thia-1-azabicyclo[3.2.0]hept-2-ene-2-carboxylic acid 1-(isopropoxycarbonyloxy)ethyl ester,

(+)-(5R, 6S)-6-[(R]-1-hydroxyethyl]-3-(3-pyridyl)-7-oxo-4-thia-1-azabicyclo[3.2.0]hept-2-ene-2-carboxylic acid isopropoxycarbonyloxymethyl ester.

The compound (I) has the same antibacterial spectrum as that described with respect to the above pivaloyloxymethyl ester and can be administered orally according to the same manner as described above. Further, they can be formulated in a pharmaceutical composition according to the same manner as that described with respect to the above pivaloyloxymethyl ester.

The following Reference Examples, Examples and Experiment further illustrate the present invention in detail but are not to be construed to limit the scope thereof.

The symbols used in the Reference Examples and Examples mean as follows:
s: singlet, br: broad, d: doublet, q: quartet, m: multiplet, ABq: AB type quartet, $CDCl_3$: deuterio chloroform, DMSO-$d_6$: dimethylsulfoxide-$d_6$, $D_2O$: heavy water and %: % by weight.

Reference Example 1

The silver salt (6.96 g) of 2-[(3S, 4R)-3-[(R)-1-allyloxycarbonyloxyethyl]-4-mercapto-2-oxoazetidin-1-yl]-2-triphenylphosphoranilidene acetic acid allyl ester was dissolved in methylene chloride (110 ml), to which were added pyridine (3.2 ml), 4-dimethylaminopyridine (200 mg) and then nicotinoyl chloride hydrochloride (2.68 g) at 0°C, and the mixture was stirred for 20 minutes. The solid formed was filtered off and the filtrate was washed in turn with sodium bicarbonate and saturated sodium chloride solution. After drying over anhydrous magnesium sulfate, the solvent was distilled off and the residue was subjected to a silica gel chromatography by eluting with toluene-ethyl acetate (9 : 1 to 3 : 2) to obtain 2-[(3S, 4R)-3-[(R)-1-allyloxycarbonyloxyethyl]-4-nicotinoylthio-2-oxoazetidinyl]-2-triphenylphosphoranilideneacetic acid allyl ester [TLC: $R_f = 0.33$ (silica gel, ethyl acetate)].

The ester of the penem compound thus obtained (3.1 g) was dissolved in toluene (600 ml) and the mixture was stirred at the reflux temperature for 3 hours under argon atmosphere. The solvent was evaporated and the residue was subjected to a silica gel chromatography by eluting with toluene-ethyl acetate (9 : 1 to 85 : 15) to obtain (5R, 6S)-6-[(R)-1-allyloxycarbonyloxyethyl]-3-(3-pyridyl)-7-oxo-4-thia-1-azabicyclo[3.2.0]hept-2-ene-carboxylic acid allyl ester [TLC: $R_f = 0.5$ (silica gel, ethyl acetate)].

The allyl ester (1.1 g) of the penem compound thus obtained was dissolved in tetrahydrofuran (48 ml) and to this were added tetrakis(triphenylphosphine)palladium (111 mg) and tributyl tin hydride (1.6 ml). After stirring at room temperature for 35 minutes, acetic acid (0.36 ml) was added dropwise. The mixture was further stirred for 30 minutes and concentrated by a rotary evaporator. The residue was added to water-ethyl acetate and then neutralized with sodium bicarbonate. The aqueous layer was washed twice with ethyl acetate and concentrated under vacuum. Then, it was subjected to a chromatography on OptiUPC$_{12}$ by eluting with water to obtain the corresponding sodium salt [TLC: $R_f = 0.61$ (OptiUPC$_{12}$, water)].

The sodium salt thus obtained (62 g) was dissolved in dimethyl formamide (1.5 ml). After cooling to 0°C, methylpivalate iodide (53 µl) was added. After stirring for one hour, the reaction mixture was diluted with ethyl acetate, washed three times with a saturated sodium chloride solution, dried and then concentrated. The residue was subjected to a silica gel chromatography by eluting with toluene-ethyl acetate (3 : 1 to 1 : 1) to obtain the corresponding pivaloyloxymethyl ester [TLC: $R_f = 0.34$ (silica gel, ethyl acetate)].

This product was a hygroscopic powder and it was confirmed to be amorphous by X-ray diffraction.

Reference Example 2

Potassium hydroxide (67.3 g) was suspended in tetrahydrofuran (1.2 liters) and to the suspension was added 2-ethylhexanoic acid (173.1 g) was added. The mixture was stirred until potassium hydroxide was dissolved to obtain a solution of potassium 2-ethylhexanoate.

(+)-(5R, 6S)-6-[(R)-1-Hydroxyethyl]-3-(3-pyridyl)-7-oxo-4-thia-1-azabicyclo[3.2.0]hept-2-ene-2-carboxylic acid allyl ester (332.4 g) was dissolved in a mixture of methylene chloride (2.5 liters) and tetrahydrofuran (1.3 liters). To the solution were added triphenylphosphine (26.2 g) and tetrakis(triphenylphosphine)-palladium (11.6 g), followed by further addition of the above solution of potassium 2-ethylhexanoate. The mixture was stirred for 15 minutes and ethyl ether was added dropwise thereto to deposit crystals. After stirring for 30 minutes, the crystals were collected by filtration and washed with ethyl ether (2 liters). The crystals were dried under reduced pressure to obtain crude crystals of potassium (+)-(5R, 6S)-6-[(R)-1-hydroxyethyl]-3-(3-pyridyl)-7-oxo-4-thia-1-azabicyclo[3.2.0]hept-2-ene-2-carboxylate.

The crude crystals were dissolved in a mixture methanol (460 ml) and water (230 ml) and to the solution was added dropwise acetonitrile (7.0 liters) to deposit crystals. The mixture was cooled to 10°C or lower and allowed to stand for 1 hour. The crystals were collected by filtration and washed with acetonitrile (3.5 liters) and dried under reduced pressure to obtain crystals of potassium (+)-(5R, 6S)-6-[(R)-1-hydroxyethyl]-3-(3-pyridyl)-7-oxo-4-thia-1-azabicyclo[3.2.0]hept-2-ene-2-carboxylate.

The diffraction patter by powder X-ray diffraction pattern is shown in Fig. 2. As seen from Fig. 2, the crystalline potassium salt has a diffraction pattern which shows main peaks at spacing of 13.6, 11.2, 4.10, 4.05 and 3.7 Å.

Example 1

An amorphous powder of (+)-(5R, 6S)-6-[(R)-1-hydroxyethyl]-3-(3-pyridyl)-7-oxo-4-thia-1-azabicyclo-[3.2.0]hept-2-ene-carboxylic acid pivaloyloxymethyl ester (19.5 g) obtained in the above Reference Example 1 was dissolved in ethanol (98 ml). This solution was heated to 31°C, followed by the addition of water which had been heated to 32°C. To the mixture was added an activated charcoal (Shirasagi P, manufactured by Takeda Chemical Industries, Ltd., 0.98 g) and the mixture was stirred for 10 minutes. Then, the activated charcoal was removed and washed with a mixed solvent of ethanol (20 ml) and water (29 ml). The filtrate was stirred at 25 to 30°C for one hour, cooled to 10°C and further stirred for one hour. The crystals deposited were filtered off, washed in turn with a mixed solvent of ethanol (20 ml) and water (39 ml) and further water (120 ml), and then dried under reduced pressure at 35°C for 5 minutes to obtain crystals (16.6 g) of the ester as white powder.

Melting point: 95-96°C

Water content (Karl Ficher's method): 0.02 %

Ethanol content (measured by gas chromatography): 0.03 %

Powder X-ray diffraction pattern: The diffraction pattern is shown in Fig. 1.

Example 2

The amorphous powder (1.18 g) of the ester obtained in the above Reference Example 1 was dissolved in ethyl acetate (12 ml), to which was added n-hexane (24 ml) with stirring. The solution thus obtained was stirred at 25°C for 20 minutes, cooled at 5°C and then allowed to stand for one hour. The crystals deposited were filtered off, washed with ethyl alcohol (9 ml) and then dried under reduced pressure to obtain crystals (0.84 g) of the ester.

Melting point: 95.5-96°C

IR (KBr method): 1766, 1714, 1320, 1138, 1106, 982 $cm^{-1}$

NMR ($CDCl_3$): $\delta$ 1.16 (9H, s), 1.38 (3H, d, J=6Hz), 3.84 (1H, dd, J=1.5x6Hz), 4.28 (1H, quint., J=6Hz), 5.66, 5.77 (2H, ABq, J=5.5Hz), 5.77 (1H, d, J=1.5Hz), 7.2-8.7 (4H, m) ppm

Powder X-ray diffraction pattern: It showed the same characteristic peaks as those obtained in Example 1.

Example 3

A diluted ethyl acetate solution containing the ester (1530 g) obtained in the above Reference Example 1 was concentrated under reduced pressure till it became a viscose oily state. To the oily product was added ethyl ether (4.5 liters) and the mixture was stirred at 25°C for 30 minutes and allowed to stand at 5°C for 16 hours. The crystals deposited were filtered off, washed with ethyl ether (4.5 liters) and then dried under reduced pressure at 35°C for about 7 hours to obtain crystals (the first crop of the crystals, 1131 g) of the ester.

Melting point: 96-96.5°C

The filtrate was concentrated under reduced pressure and to the resulting oily product (about 600 g) was added ethyl ether (1.2 liters), followed by stirring at 25°C for 1.5 hours and further stirring at 50°C for 30 minutes. The crystals deposited were filtered off, washed with ethyl ether (1.2 liters) and then dried under reduced pressure at 35°C for about 3 hours to obtain crystals (the second crop of the crystals, 335 g) of the ester.

Powder X-ray diffraction pattern: Both first and second crops showed the same characteristic peaks as those in Example 1.

Example 4

According to the following formulation, 50,000 tablets were prepared.

| Components | in one tablet (mg) | in 50,000 tablets (kg) |
|---|---|---|
| The crystals of the penem compound | 139 | 6.95 |
| Lactose | 28 | 1.4 |
| Corn starch | 26.4 | 1.32 |
| Hydroxypropyl cellulose | 6 | 0.3 |
| Magnesium stearate | 0.6 | 0.03 |
| Total | 200 | 10 |

The crystals of the penem compound of the present invention (6.95 kg), lactose (1.4 kg) and a part of corn starch (1 kg) were mixed in a fluidized pelletizer (FD-S-2, manufactured by Pawleck) and then 6% aqueous solution of hydroxypropyl cellulose (5 kg, corresponding to 0.3 kg of hydroxypropyl cellulose) was sprayed to obtain pellets. To the pellets thus obtained were added corn starch (0.32 kg) and magnesium stearate (0.03 kg) and the mixture was thoroughly mixed. The mixture was compressed by a rotary tabletting machine (clean press manufactured by Kikusui K.K., Japan) to obtain about 50,000 tablets of 8 mm in diameter.

Example 5

Potassium (+)-(5R, 6S)-6-[(R)-1-hydroxyethyl]-3-(3-pyridyl)-7-oxo-4-thia-1-azabicyclo[3.2.0]hept-2-ene-2-carboxylate (7.11 g) obtained in Reference Example 2 was dissolved in N,N-dimethylacetamide (100 ml) and the solution was cooled to -15°C. To the solution was added cyclohexyl-1-iodoethyl-carbonate(8.99 g) and the mixture was stirred at -10°C for 30 minutes. To the mixture were added 5% (w/v) aqueous sodium thiosulfate solution (120 ml) and ethyl acetate (50 ml) to separete into layers and the aqueous layer was extracted with ethyl acetate (50 ml). The organic layers were combined and washed twice with saturated

saline (120 ml) and dried over anhydrous magnesium sulfate (20 g). After concentration under reduced pressure, the residue was chromatographed on a silica gel column and eluted with ethyl acetate. The eluate was concentrated under reduced pressure to obtain a powder of (+)-(5R, 6S)-6-[(R)-1-hydroxyethyl]-3-(3-pyridyl)-7-oxo-4-thia-1-azabicyclo-[3.2.0]hept-2-ene-2-carboxylic acid 1-(cyclohexyloxycarbonyloxy)ethyl ester.

NMR ($CDCl_3$): δ 1.0-2.2 (10H, m), 1.38 (3H, d, J=6Hz), 2.16 (3H, s), 2.63 (1H, br s), 3.84 (1H, dd, J=1.5x6.8Hz), 4.1-4.5 (1H, m), 4.4-4.9 (1H, m), 5.78 (1H, d, J=1.5Hz), 6.72 (1H, q, J=5.5Hz), 7.2-8.8 (4H, m) ppm

IR (KBr method): 2980, 1810, 1775, 1300, 1280, 1100 $cm^{-1}$

Experiment

The crystalline powder of the ester produced according to the process of the present invention and the amorphous powder produced in the Reference Example 1 were stored at a dark place in a sealed container at a temperature of 60°C, respectively, and the residual rate was measured. The results are shown in Table 1 below.

Table 1

| Sample | Conditions of storage | Residual rate |
|---|---|---|
| Amorphous powder | 60°C, 14 days | 37.7 % |
| Crystalline powder of Example 1 | 60°C, 19 days | 98.7 % |
| Crystalline powder of Example 2 | 60°C, 14 days | 98.9 % |

## Claims

1. Crystalline (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester having a diffraction pattern which shows main peaks at spacing of 12.8, 8.8, 5.6, 4.44, 4.36, and 4.2 Å according to powder X-ray diffraction.

2. A process for producing a crystalline penem compound which comprising dissolving amorphous (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester in a good solvent, adding thereto a poor solvent which is miscible with the good solvent, stirring the mixture and then cooling to a temperature of not higher than 30°C to obtain crystalline (+)-(5R, 6S)-6-[(R)-1-hydroxyethyl]-3-(3-pyridyl)-7-oxo-4-thia-1-azabicyclo[3.2.0]hept-2-ene-2-carboxylic acid pivaloyloxymethyl ester having a diffraction pattern which shows main peaks at spacing of 12.8, 8.8, 5.6, 4.44, 4.36, and 4.2 Å according to powder X-ray diffraction.

3. A process according to claim 2, wherein the good solvent is selected from lower alcohols or esters.

4. A process according to claim 3, wherein the lower alcohol is methanol, ethanol, propanol or isopropanol.

5. A process according to claim 3, wherein the ester is ethyl acetate.

6. A process according to claim 2, wherein the poor solvent is water in the case of using a lower alcohol as the good solvent.

7. A process according to claim 2, wherein the poor solvent is ethyl ether, n-hexane or petroleum ether in the case of using an ester as the good solvent.

8. An antibacterial agent which comprises the crystalline penem compound of claim 1.

9. Use of the crystalline penem compound of claim 1 in the preparation of an antibacterial composition.

10. Crystalline potassium (+)-(5R, 6S)-6-[(R)-1-hydroxyethyl]-3-(3-pyridyl)-7-oxo-4-thia-1-azabicyclo-[3.2.0]-hept-2-ene-2-carboxylate having a diffraction pattern which shows main peaks at spacing of 13.6, 11.2, 4.10. 4.05 and 3.7 Å according to powder X-ray diffraction.

Fig. 1

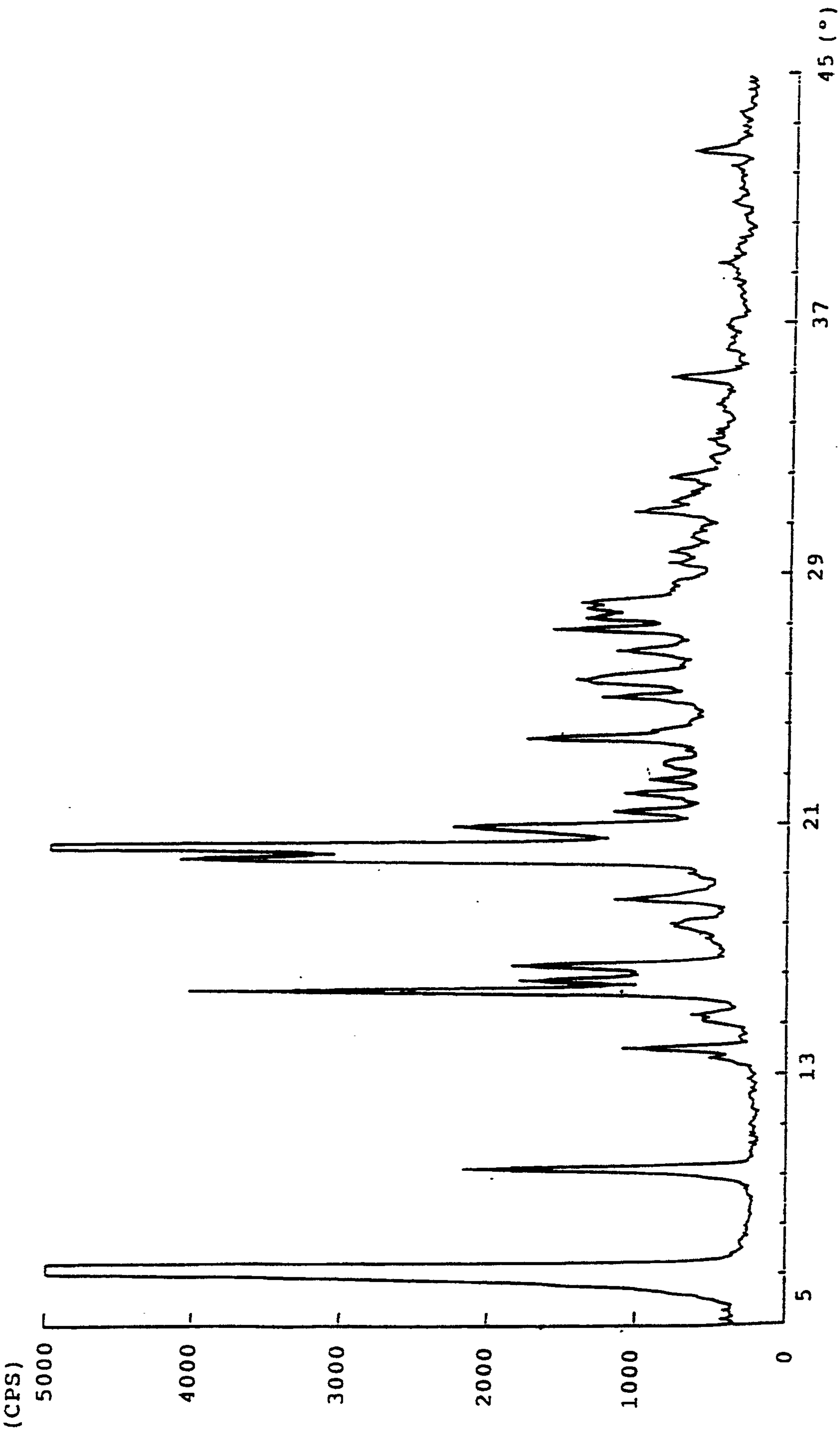

Fig. 2

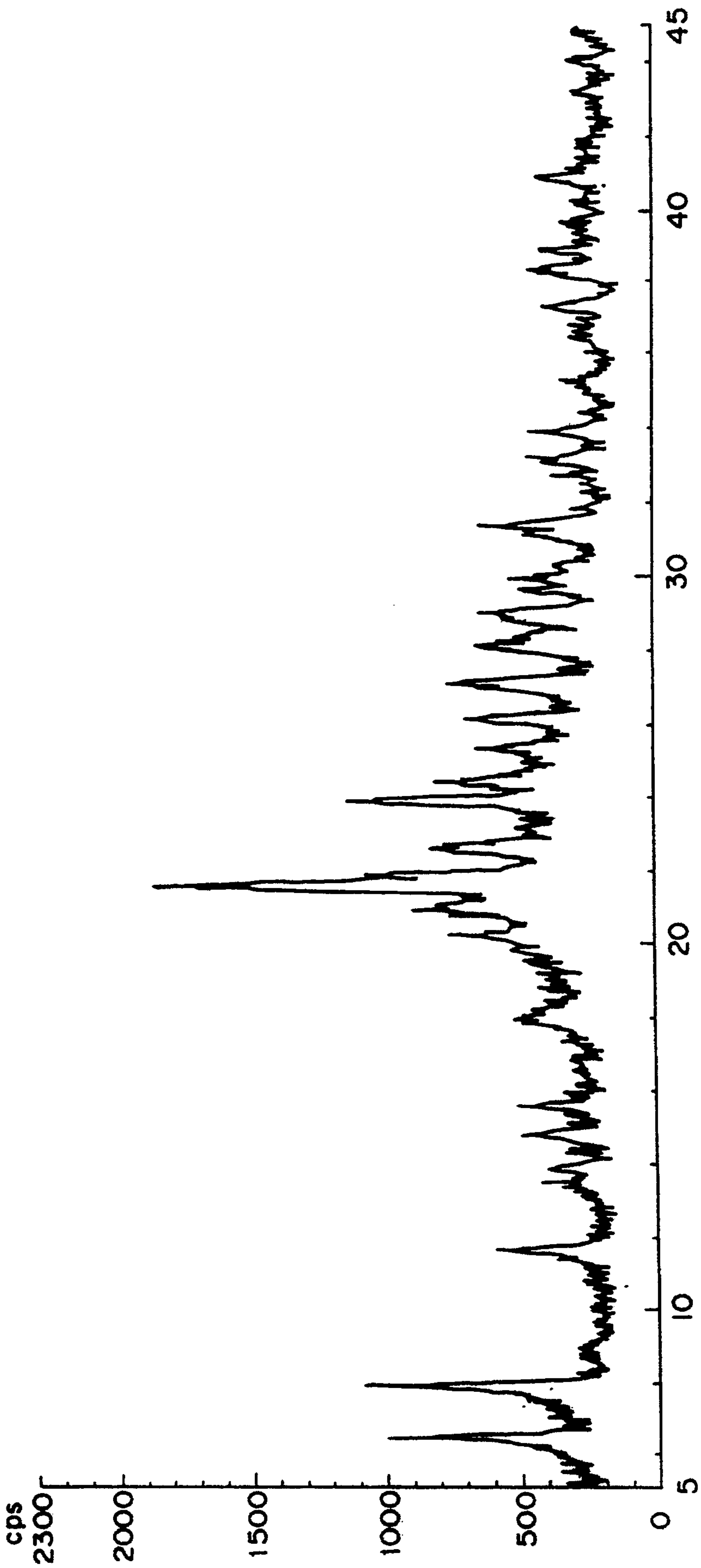

European Patent Office

# EUROPEAN SEARCH REPORT

Application Number

**EP 91 10 9784**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | THE JOURNAL OF ANTIBIOTICS, vol. 43, no. 3, March 1990, pages 306-313, Japan Antibiotics Research Association, Tokyo, JP; A. BEDESCHI et al.: "Synthesis and structure-activity relations in the class of 2-(pyridyl)penems" * The whole article; especially page 307, formula 4n; page 311, last paragraph * | 1-10 | C 07 D 499/88<br>A 61 K 31/43 |
| A | CHEMICAL ABSTRACTS, vol. 97, no. 5, 2nd August 1982, page 555, abstract no. 38753p, Columbus, Ohio, US; & CS-A-197 138 (L. NOVAK) 30-04-1982 * Abstract * | 1-10 | |
| D,A | EP-A-0 246 187 (CIBA-GEIGY AG) * Page 31, example 7; claims * | 1-10 | |
| | | | **TECHNICAL FIELDS SEARCHED (Int. Cl.5)** |
| | | | C 07 D 499/00<br>A 61 K 31/00 |

The present search report has been drawn up for all claims

| Place of search | Date of completion of search | Examiner |
|---|---|---|
| The Hague | 23 September 91 | CHOULY J. |

**CATEGORY OF CITED DOCUMENTS**

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document